# EUROPÄISCHE PATENTSCHRIFT

(11) **EP 1 252 134 B1**
(45) Veröffentlichungstag und Bekanntmachung des Hinweises auf die Patenterteilung: **06.10.2004**
(21) Anmeldenummer: 01913783.5
(22) Anmeldetag: 02.02.2001
(51) Int. Cl.: C07C 231/04, C07C 237/16, C07C 237/10, C07C 231/12

(54) **VERFAHREN ZUR HERSTELLUNG VON ACETOACETYLIERTEN AROMATISCHEN AMINEN**
METHOD FOR PRODUCING ACETOACETYLATED AROMATIC AMINES
PROCEDE DE PREPARATION D'AMINES AROMATIQUES ACETOACETYLEES

(30) Priorität: 04.02.2000 EP 00102418; 12.05.2000 US 203922 P
(43) Veröffentlichungstag der Anmeldung: 30.10.2002
(73) Patentinhaber: Lonza AG, 4052 Basel (CH)
(72) Erfinder: GLUFKE, Uta, CH-5054 Basel (CH); HANSELMANN, Paul, CH-3902 Brig-Glis (CH)
(86) Internationale Anmeldenummer: PCT/EP2001/001163
(87) Internationale Veröffentlichungsnummer: WO 2001/056973

(56) Entgegenhaltungen:
- EP-A- 0 719 762
- US-A- 3 702 365
- US-A- 5 466 268
- DATABASE CHEMABS [Online] CHEMICAL ABSTRACTS SERVICE, COLUMBUS, OHIO, US; THIELE, KURT: "N-Aromatic substituted acid amides as analgesics" retrieved from STN Database accession no. 70:68180 XP002171561 & ZA 6 706 852 1 (DEUTSCHE GOLD- UND SILBER-SCHEIDEANSTALT VORM. ROESSLER) 27. Juni 1968 (1968-06-27)

## Beschreibung

Die vorliegende Erfindung betrifft ein Verfahren zur Herstellung von acetoacetylierten aromatischen Aminen, insbesondere von acetoacetylierten *N*-Phenyl-*p*-phenylendiaminen sowie neue acetoacetylierte *N*-Phenyl-*p*-phenylendiamine.

US Patent Nr. 2 115 413 beschreibt die Umsetzung von Acetessigsäureethylester mit N-Phenyl-*p*-phenylendiamin zur Herstellung von 3-Oxo-*N*-[4-(phenylamino)-phenyl]butyramid.

Die Umsetzung von primären aromatischen Aminen mit Diketen zur Herstellung von Acetoacetaniliden beschreiben C. E. Kaslow et al. in *J. Am. Chem. Soc.* **1946**, *68*, 644-647.

Die Umsetzung von sekundären aromatischen Aminen mit Diketen zur Herstellung von *N*-Alkylacetoacetaniliden beschreiben C. E. Kaslow et al. in *J Am. Chem. Soc.* **1945,** *67*,1969-1970. Die Umsetzung wird auch von N. Etkin et al. in *J. Org. Chem.* **1990**, *55*, 1093-1096 beschrieben. Die hierbei eingesetzten sekundären aromatischen Amine sind alkyl- oder alkoxysubstituierte Aniline. Die Umsetzung von *N*-C₁₋₆-Alkyl-3-oxo-*N*-[4-(phenylamino)phenyl]butyramiden wird nicht beschrieben.

US-A 3 702 365 beschreibt die Umsetzung von 4-Aminodiphenylamin mit Diketen in Dioxan als Lösungsmittel.

Aufgabe der vorliegenden Erfindung war es, ein Verfahren zur Herstellung von acetoacetylierten *N*-Phenyl-*p*-phenylendiaminen bereitzustellen.

Diese Aufgabe wird durch das Verfahren gemäss Patentanspruch 1 gelöst.

Das Verfahren betrifft die Herstellung von Verbindungen der allgemeinen Formel 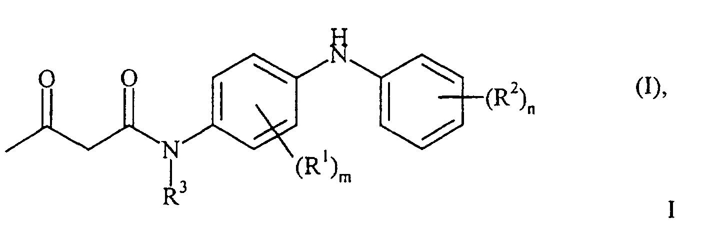 worin
- R¹ und R²: bei jedem Auftreten unabhängig voneinander jeweils Hydroxy, C₁₋₆-Alkyl, C₁₋₆-Alkoxy, Halogen, Phenyl oder Phenoxy;
- R³: Wasserstoff oder C₁₋₆-Alkyl;
- m: eine ganze Zahl von 0 bis 4; und
- n: eine ganze Zahl von 0 bis 5 bedeuten.

Diese Verbindungen können in mindestens zwei tautomeren Formen (Keto/Enol-Tautomerie) vorliegen, wobei hier zur Vereinfachung jeweils nur die Ketoform abgebildet ist. Die Erfindung umfasst jedoch alle Tautomeren und deren Gemische.

Das Verfahren ist dadurch gekennzeichnet, dass Diketen mit einem *N*-Phenyl-*p*-phenylendiamin der allgemeinen Formel 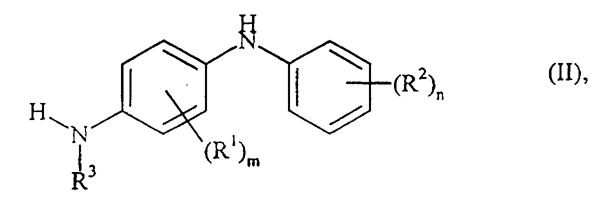 worin R¹, R², R³, m und n die oben angegebene Bedeutung haben, in Gegenwart von 3-40%iger Essigsäure bei Temperaturen von 20 bis 100 °C, vorzugsweise bei 60 bis 70 °C umgesetzt wird.

Unter C₁₋₆-Alkyl sind hier und im folgenden alle linearen oder verzweigten Alkylgruppen mit 1-6 Kohlenstoffatomen zu verstehen, wie z. B. Methyl, Ethyl, Propyl, Isopropyl, Butyl, Isobutyl, *sec*-Butyl, *tert-*Butyl, Pentyl, Isopentyl, *tert*-Pentyl, Neopentyl, Hexyl oder Isohexyl.

Unter C₁₋₆-Alkoxy sind Gruppen, die aus C₁₋₆-Alkyl und Sauerstoff zusammengesetzt sind, zu verstehen, wie z. B. Methoxy, Ethoxy, Propoxy, Isopropoxy, Butoxy, *sec*-Butoxy oder *tert*-Butoxy.

Die Variablen m und n haben vorzugsweise den Wert 0 (Null). Der Rest R³ ist vorzugsweise C₁₋₆-Alkyl, besonders bevorzugt Isopropyl.

Überraschenderweise erfolgt die Reaktion des *N*-Phenyl-*p*-phenylendiamins (II) mit Diketen nur am Stickstoffatom, welches den Rest R³ trägt.

Die Verbindung der Formel II, worin m = n = 0 ist und R³ Wasserstoff bedeutet, ist im Handel erhältlich (z. B. bei der Firma Fluka), die übrigen Verbindungen können nach bekannten Methoden, z. B. durch Umsetzung von Anilin oder substituierten Anilinen mit Nitrobenzol oder Azobenzol und anschliessende Hydrierung, hergestellt werden. Die Verbindungen der Formel II, worin R³ C₁₋₆-Alkyl bedeutet, können nach bekannten Methoden, z. B. durch reduktive Alkylierung von *N*-Phenyl-*p*-phenylendiaminen der Formel II (R³ = H) mit einem aliphatischen Keton oder Aldehyd hergestellt werden.

Die Verbindungen der Formel I, worin R³ C₁₋₆-Alkyl bedeutet, sind neu und ebenfalls Gegenstand der Erfindung. Diese Verbindungen können beispielsweise als Additive für Kraftstoffe von Verbrennungsmotoren eingesetzt werden. Eine weitere Einsatzmöglichkeit ist die als Trocknungsbeschleuniger für Polymere.

Die Verbindungen der Formel I können durch Umsetzung mit Ammoniak in die entsprechenden Enamine der allgemeinen Formel 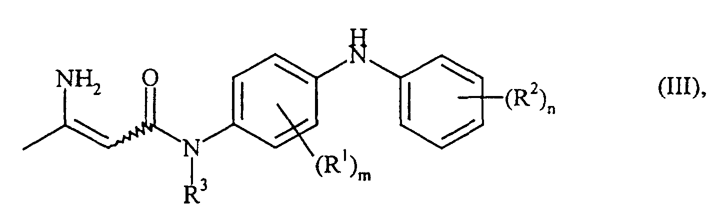 worin R¹, R², R³, m und n die oben angegebene Bedeutung haben, umgewandelt werden. Diese Enamine können in zwei geometrischen Isomeren (*E* und *Z*-Konfiguration an der Enamin-Doppelbindung) vorliegen, wobei hier sowohl die beiden Isomeren als auch deren Gemische umfasst sind. Vorzugsweise wird die Z-Konfiguration gebildet.

Die Umsetzung mit Ammoniak erfolgt vorteilhaft bei Temperaturen von 10 bis 150°C, vorzugsweise etwa 70 °C, und Drücken von 1 bis 100 bar, vorzugsweise 10 bis 30 bar. Die Umsetzung erfolgt vorteilhaft in einem geeigneten Lösungsmittel in Gegenwart von katalytischen Mengen an konzentrierter oder wässriger Essigsäure. Geeignete Lösungsmittel sind beispielsweise Ester,.aromatische und aliphatische Kohlenwasserstoffe, chlorierte aliphatische Kohlenwasserstoffe, Ether und Polyether, Alkohole sowie Wasser. Bevorzugt wird Ethylacetat als Lösungsmittel eingesetzt. Vorteilhaft werden 0,02-2,0 mol konzentrierte Essigsäure auf 1 mol der Verbindung der Formel I eingesetzt.

Die Verbindungen der Formel III, worin R³ C₁₋₆-Alkyl bedeutet, sind neu.

Durch katalytische Hydrierung der Verbindungen der Formel III entstehen Verbindungen der allgemeinen Formel 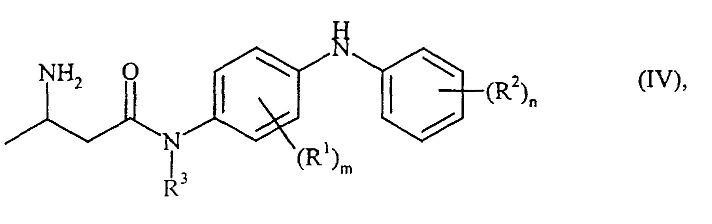 worin R¹, R², R³, m und n die oben angegebene Bedeutung haben.

Die katalytische Hydrierung erfolgt vorteilhaft bei Temperaturen von 100 bis 200 °C, vorzugsweise etwa 110 °C, und Drücken von 20 bis 150 bar, vorzugsweise etwa 100 bar. Ein geeigneter Hydrierkatalysator ist beispielsweise Raney-Nickel. Die Hydrierung erfolgt vorteilhaft in einem geeigneten Lösungsmittel in Gegenwart einer Base, vorzugsweise in Gegenwart von Ammoniak. Geeignete Lösungsmittel sind beispielsweise aliphatische Alkohole oder Mischungen von Wasser und aliphatischen Alkoholen, bevorzugt wird Methanol eingesetzt.

Die Verbindungen der Formel IV, worin R³ C₁₋₆-Alkyl bedeutet, sind neu.

Die folgenden Beispiele verdeutlichen die Durchführung des erfindungsgemässen Verfahrens und die Herstellung der erfindungsgemässen Verbindungen, ohne dass darin eine Einschränkung zu sehen ist.

### Beispiel 1

### 3-Oxo-N-[4-(phenylamino)-phenyl]butyramid (I, m = n = 0, R³ = H)

### A) simultane Zugabe der Edukte

Diketen (160,13 g, 1,904 mol) sowie eine Lösung von *N*-Phenyl-*p*-phenylendiamin (344,00 g, 1,867 mol) in 40%iger Essigsäure (1300,00 g) wurden simultan während circa 4,5 h zu 40%iger Essigsäure (300,00 g) bei 60 °C zugetropft. Nach erfolgter Zugabe der Edukte wurde das Reaktionsgemisch 30 min bei 65 °C gerührt und dann auf 15 °C abgekühlt. Der entstandene Niederschlag wurde abgenutscht, mit 40%iger Essigsäure (2×200 g) sowie mit Wasser (2×200 g) gewaschen und im Vakuum (20 mbar) bei 50 °C getrocknet. Man erhielt 415,31 g (95,3% Gehalt, 79% Ausbeute) 3-Oxo-*N*-[4-(phenylamino)-phenyl]butyramid als hellgrauen Feststoff. Mp: 89,5 °C;
¹H NMR (400 MHz, DMSO-d₆) δ = 13,89 (s, 0,1 H, O*H*_{Enol}); 9,93 (s, 0,9 H, N*H*_{Keto}); 9,87 (s, 0,1 H, N*H*_{Enol}); 8,02 (s, 1 H, N*H*); 7,50-7,42 (m, 2 H, Ar-H); 7,25-7,16 (m, 2 H, Ar-*H*); 7,10-6,98 (m, 4 H, Ar-*H*); 6,80-6,70 (m, 1 H, Ar-*H*); 5,18 (s, 0,1 H, CO-C*H*=COH); 3,53 (s, 1,9 H, CO-C*H*₂-CO); 2,12 (s, 2,7 H, C*H*_{3 Keto}); 1,90 (s, 0,3 H, C*H*_{3 Enol}).

### B) nicht simultane Zugabe der Edukte

Diketen (18,6 g, 0,221 mol) wurde zu einer Lösung von *N*-Phenyl-*p*-phenylendiamin (40,00 g, 0,217 mol) in 40%iger Essigsäure (100,00 g) während 40 min bei 60 °C zugetropft. Das Reaktionsgemisch wurde 30 min bei 65 °C gerührt und dann auf 15°C abgekühlt. Die sehr dickflüssige Suspension wurde filtriert. Der Niederschlag wurde mit 40%iger Essigsäure (2×25 g) sowie Wasser (2×25 g) gewaschen und im Vakuum (20 mbar) bei 50 °C getrocknet. Man erhielt 53,50 g (92% Ausbeute) 3-Oxo-*N*-[4-(phenylamino)phenyl]butyramid als hellgrauen Feststoff.
Mp: 93,0 °C.

### Beispiel 2

### N-Isopropyl-3-oxo-N-[4-(phenylamino)phenyl]butyramid (I, m = n = 0, R³ = Isopropyl)

Diketen (261,10 g, 3,105 mol) wurde zu einer Lösung vom *N*-(Isopropylamino)-*N*-phenyl*p*-phenylendiamin (388,00 g, 1,714 mol) in 40%iger Essigsäure (1200 ml) während 2 h bei 60°C zugetropft. Nach der Zugabe des Diketens wurde das Reaktionsgemisch 2,5 h bei 65 °C gerührt und über Nacht auf 5 °C gekühlt. Der entstandene Niederschlag wurde abgenutscht, mit Wasser (2×150 ml) gewaschen und im Vakuum (20 mbar) bei 40 °C getrocknet. Man erhielt 400,60 g (75% Ausbeute) *N*-Isopropyl-3-oxo-*N*-[4-(phenylamino)phenyl]butyramid als dunkelgrauen Feststoff.
¹H NMR (400 MHz, CDCl₃) δ = 14,48 (s, 0,1 H, O*H*_{Enol}); 7,35-7,25 (m, 2 H, Ar-*H*); 7,20-7,10 (m, 2 H, Ar-*H*); 7,10-6,95 (m, 3 H, Ar-*H*); 6,95-6,90 (m, 2 H, Ar-*H*); 6,01 (s, 1 H, N*H*); 5,05-4,92 (m, 1 H, C*H*(CH₃)₂); 4,45 (s, 0,1 H, CO-C*H*=COH); 3,21 (s, 1,9 H, CO-C*H*₂-CO); 2,10 (s, 2,7 H, C*H*_{3 Keto}); 1,89 (s, 0,3 H, *CH*_{3 Enol}); 1,09 (d, *J* = 8,2 Hz, 6 H, CH(*CH*₃)₂).

### Beispiel 3

### (Z)-3-Aminobut-2-ensäure-N-isopropyl-N-[4-(phenylamino)phenyl]amid (III, m = n = 0, R³ = Isopropyl)

In einem Autoklaven wurden *N*-Isopropyl-3-oxo-*N*-[4-(phenylamino)phenyl]butyramid (30,0 g, 0,0967 mol) und konz. Essigsäure (1,4 g, 0,0233 mol) in Ethylacetat (140 g) vorgelegt. Innerhalb von 10 min wurde Ammoniak (16,0 g, 0,9395 mol) eingeleitet und die Temperatur des Reaktionsgemisches während dieser Zeit langsam auf 35 °C erhöht. Nach dem Einleiten betrug der Druck 14 bar. Das Reaktionsgemisch wurde dann innerhalb von 30 min auf 70 °C erhitzt, wobei der Druck auf 17 bar stieg. Nach weiteren 2,5 h unter diesen Bedingungen wurde das Reaktionsgemisch auf 25 °C abgekühlt und das überschüssige Ammoniak abgelassen. Das Reaktionsgemisch wurde weiter auf 5 °C gekühlt, um das Produkt zu kristallisieren. Die Suspension wurde filtriert und der erhaltene hellgraue Feststoff mit 0 °C kaltem Ethylacetat (20 ml) gewaschen und 2 Tage bei 35 °C im Vakuum (50 mbar) getrocknet. Man erhält (*Z*)-3-Aminobut-2-ensäure-*N*-isopropyl-*N*-[4-(phenylamino)phenyl]amid (27,2 g, 91,0%).
¹H NMR (400 MHz, CDCl₃) δ = 7,36-7,25 (m, 2 H, Ar-*H*); 7,18-7,11 (m, 2 H, Ar-*H*); 7,09-7,02 (m, 2 H, Ar-*H*); 6,99-6,96 (m, 3 H, Ar-*H*); 5,84 (s, 1 H, N*H*); 5,05-4,99 (m, 1H, C*H*(CH₃)₂); 4,04 (s, 1 H, CO-C*H*=C); 1,71 (s, 3 H, C*H*₃); 1,03 (d, *J*= 8 Hz, 6 H, CH(C*H*₃)₂).

### Beispiel 4

### 3-Amino-N-isopropyl-N-[4-(phenylamino)phenyl]butyramid (IV, m = n = 0, R³ = Isopropyl)

In einem Autoklaven wurden (*Z*)-3-Aminobut-2-ensäure-*N*-isopropyl-*N*-[4-(phenylamino)-phenyl]amid (13,9 g, 0,0449 mol) und Raney-Nickel (K0840 Ni B113W) (2,0 g) in Methanol (140 g) vorgelegt. Innerhalb von 5 min wurde Ammoniak (5,5 g, 0,3230 mol) eingeleitet. Die Temperatur des Reaktionsgemisches betrug 25 °C. Der Druck stieg auf 5 bar. Nach dem Einleiten von Ammoniak wurde das Reaktionsgemisch auf 80 °C erwärmt (kein weiterer Druckanstieg). Bei 80 °C wurde Wasserstoff aufgepresst, bis der Druck 100 bar betrug. Das Reaktionsgemisch wurde auf 110 °C erwärmt und während 4,5 h bei dieser Temperatur gerührt. Hierbei wurde ein Druck von 100 bar durch kontinuierliches Aufpressen von Wasserstoff aufrechterhalten. Dann wurde das Reaktionsgemisch auf 25 °C abgekühlt und der Druck vorsichtig abgelassen. Das Reaktionsgemisch wurde filtriert und das Filtrat zur Hälfte eingeeingt. Das Konzentrat wurde mit 1 g Aktivkohle versetzt, 1 h gerührt, filtriert und zur Trockne eingeengt. Man erhielt 12,8 g Rohprodukt, welches bei 175 °C geschmolzen wurde. Die Schmelze liess man auf einer Aluminiumfolie auskristallisieren. Man erhielt 11,1 g (79,3%) 3-Amino-*N*-isopropyl-*N*-[4-(phenylamino)phenyl]butyramid.
¹H NMR (400 MHz, CDCl₃) δ = 7,38-6,90 (m, 9 H, Ar-*H*); 6,03 (s, 1 H, N*H*); 5,05-4,95 (m, 1 H, C*H*(CH₃)₂); 3,42-3,32 (m, 1 H, C*H*-NH₂); 2,10-1,90 (m, 2 H, COC*H*₂); 1,53 (bs, 2 H, NH₂); 1,03 (d, *J* = 8 Hz, 6 H, CH(C*H*₃)₂); 0,98 (d, *J* = 8 Hz, 3 H, C*H*₃).

## Patentansprüche

1. Verfahren zur Herstellung von Verbindungen der allgemeinen Formel 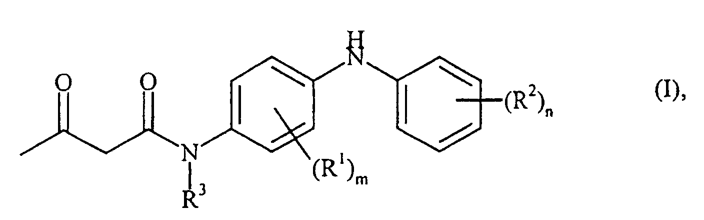 worin
R¹ und R² bei jedem Auftreten unabhängig voneinander jeweils Hydroxy, C₁₋₆-Alkyl, C₁₋₆-Alkoxy, Halogen, Phenyl oder Phenoxy;
R³ Wasserstoff oder C₁₋₆-Alkyl;
m eine ganze Zahl von 0 bis 4; und
n eine ganze Zahl von 0 bis 5 bedeuten,
**dadurch gekennzeichnet, dass** Diketen mit einem *N*-Phenyl-*p*-phenylendiamin der allgemeinen Formel 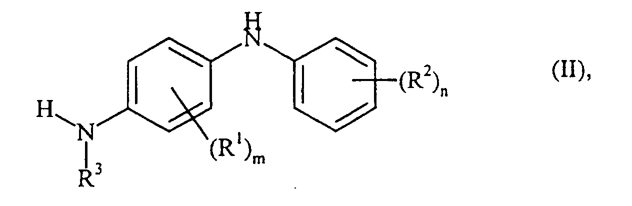 worin R¹, R², R³, m und n die oben angegebene Bedeutung haben, in Gegenwart von 3-40%iger Essigsäure bei Temperaturen von 20 bis 100 °C, vorzugsweise bei 60 bis 70 °C umgesetzt wird.

2. Verfahren nach Anspruch 1, wobei R³ C₁₋₆-Alkyl bedeutet.

3. Verbindungen der allgemeinen Formel 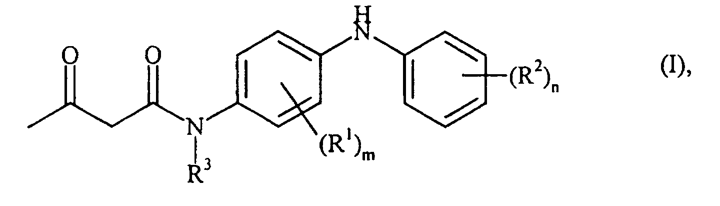 worin R¹, R², m und n die in Anspruch 1 angegebene Bedeutung haben und R³ C₁₋₆-Alkyl bedeutet.

## Claims

1. A process for the production of compounds of the general formula 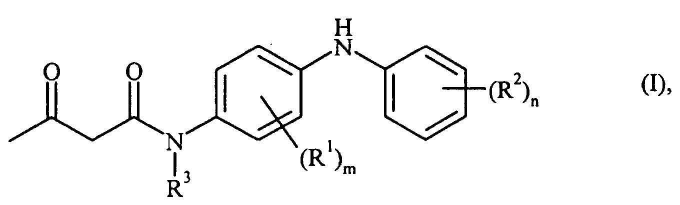 wherein
R¹ and R² at each occurrence are independently hydroxy, C₁₋₆ alkyl, C₁₋₆ alkoxy, halogen, phenyl or phenoxy;
R³ is hydrogen or C₁₋₆ alkyl;
m is an integer from 0 to 4; and
n is an integer from 0 to 5,
**characterised in that** diketene is reacted with a *N*-phenyl-*p*-phenylenediamine of the general formula 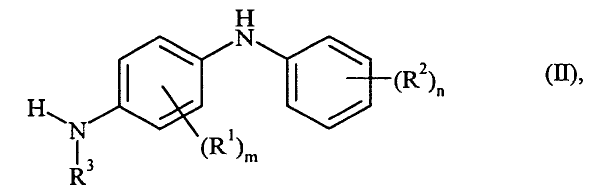 wherein R¹, R², R³, m and n have the meanings given above, in the presence of 3 to 40% strength acetic acid at temperatures from 20 to 100 °C, preferably at 60 to 70°C.

2. The process of claim 1, wherein R³ is C₁₋₆ alkyl.

3. Compounds of the general formula 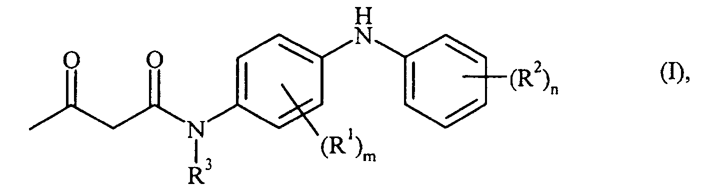 wherein R¹, R², m and n have the meanings given in claim 1 and R³ is C₁₋₆ alkyl.

## Revendications

1. Procédé de préparation de composés de la formule générale 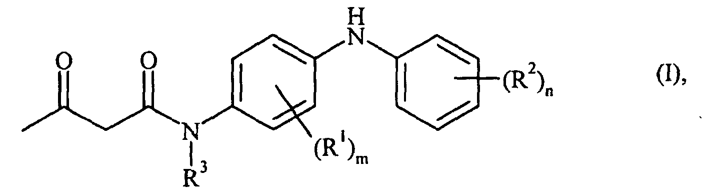 dans laquelle
R¹ et R² chaque fois représentent indépendamment l'un de l'autre l'hydroxyle, un groupe alkyle en C₁₋₆, un groupe alcoxy en C₁₋₆, un atome d'halogène, phényle ou phénoxy ;
R³ représente l'hydrogène ou un groupe alkyle en C₁₋₆ ;
m est un nombre entier de 0 à 4 ; et
n est un nombre entier de 0 à 5,
**caractérisé en ce que** le dicétène est mis en réaction avec un *N*-phényl-*p*-phénylènediamine de la formule générale 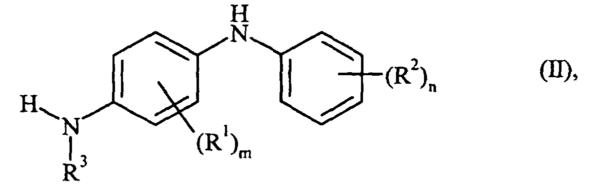 dans laquelle R¹, R², R³, m et n ont la signification citée ci-dessus, en présence d'acide acétique d'une concentration de 3 à 40% en poids et à une température de 20 à 100 °C, de préférence, de 60 à 70 °C.

2. Procédé selon la revendication 1 dans lequel R³ est un groupe alkyle en C₁₋₆.

3. Composés de la formule générale 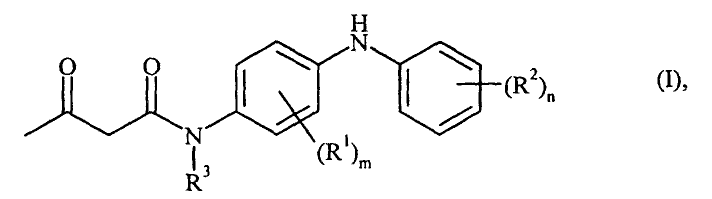 dans laquelle R¹, R², m et n ont la signification citée dans la revendication 1 et R³ est un groupe alkyle en C₁₋₆.
